# EUROPEAN PATENT APPLICATION

(11) **EP 2 508 615 A1**
(43) Date of publication of application: **10.10.2012**
(21) Application number: 10834656.0
(22) Date of filing: 03.12.2010
(51) Int. Cl.: C12P 41/00

(54) **PROCESS FOR PRODUCTION OF OPTICALLY ACTIVE AMINO ACID OR OPTICALLY ACTIVE AMINO ACID AMIDE**

(30) Priority: 04.12.2009 JP 2009276930; 04.12.2009 JP 2009276931; 04.12.2009 JP 2009276932; 04.12.2009 JP 2009276933; 04.12.2009 JP 2009276934; 04.12.2009 JP 2009276935
(71) Applicant: Mitsubishi Gas Chemical Company, Inc., Tokyo 100-8324 (JP)
(72) Inventor: IIDA Shin, Niigata-shi Niigata 950-3112 (JP); ARIE, Sachiko, Niigata-shi Niigata 950-3112 (JP); TANAKA Yusuke, Niigata-shi Niigata 950-3112 (JP)
(74) Representative: Müller-Boré & Partner Patentanwälte
(86) International application number: PCT/JP2010/071699
(87) International publication number: WO 2011/068206

(57) **Abstract**

The present invention relates to a method for producing D- or L-tert-leucine or D- or L-tert-leucine amide by reacting DL-tert-leucine amide with a biocatalyst selected from the group consisting of an enzyme capable of hydrolyzing the DL-tert-leucine amide stereoselectively, cells of a microorganism having the enzyme, a material produced by the treatment of the cells, an immobilized enzyme produced by immobilizing the enzyme onto a carrier, immobilized cells produced by immobilizing the cells onto a carrier, and an immobilized cell treatment product obtained by immobilizing the cell treatment product onto a carrier to thereby hydrolyze the DL-tert-leucine amide, wherein the hydrolysis is carried out with separating ammonia produced by the hydrolysis from the hydrolysis reaction solution. According to the present invention, the concentration of an amino acid amide as a raw material in the reaction solution can be enhanced without the need of increasing the amount of the cells or a pH-adjusting acid by maintaining the activity per unit amount of the enzyme, the cells or the cell treatment product and per unit time period at a high level in the enzymatic reaction for hydrolyzing an amino acid amide stereoselectively, and consequently the productivity of optically active tert-leucine or optically active tert-leucine amide can be improved to a great extent without increasing the generation of a waste salt or waste cells.

## Description

### CROSS-REFERENCE TO RELATED APPLICATION

This application is based upon and claims the benefit of priority from the prior Japanese Patent Application Nos. 2009-276930 filed on December 4, 2009, 2009-276931 filed on December 4, 2009, 2009-276932 filed on December 4, 2009, 2009-276933 filed on December 4, 2009, 2009-276934 filed on December 4, 2009, and 2009-276935 filed on December 4, 2009, the entire contents of which are incorporated herein by reference..

### TECHNICAL FIELD

The present invention relates to a method for preparing optically active amino acids or optically active amino acid amides. Particularly, the present invention relates to a method for efficiently preparing optically active tert-leucine or optically active tert-leucine amide.

### BACKGROUND ART

Optically active amino acids or optically active amino acid amides are the compounds important as pharmaceutical raw materials or asymmetric ligands. As the method for preparing optically active amino acids or optically active amino acid amides has been described a method for preparing an optically active amino acid or an optically active amino acid amide as an enantiomer thereof by reacting a DL-amino acid amide with an enzyme which stereoselectively hydrolyzes a D- or L-isomer selective amino acid amide (e.g., Japanese Patent Laid-Open Publication No. 63-87998).

In order to appropriately proceed enzyme reactions, it is important to carry out the reactions at a pH and temperature range within the optimal condition of an enzyme to be used, and a method for adjusting an enzyme to the optimal condition is also known in the hydrolysis reaction of an amino acid amide by an enzyme which hydrolyzes the amino acid amide stereoselectively (for example, Japanese Patent Laid-Open Publication No. 2003-225094). However, even if the reaction is started under the optimal condition as described above, the reaction rate may be lowered along with the progress of the reaction resulting in insufficient completion of the aimed reaction in the case of the high concentration of the raw materials. In order to solve such matter and to conduct successfully the enzyme reaction at higher concentrations of the raw materials, a method for adding raw materials in the repeated partial fashion (for example, Japanese Patent Laid-Open Publication No. 2005-117905) and a method for sequentially extracting a product (for example, Japanese Patent Laid-Open Publication No. H11-137286) have been described. When the problems are not solved even by these methods, it will be necessary to increase the amount of an acid for adjusting pH or to increase an enzyme. However, when the acid for adjusting pH has been increased, it is necessary to increase the reaction steps in order to neutralize the acid added. Furthermore, a large amount of the salt is produced as a by-product to be discarded along with the neutralization. In addition, a large amount of bacterial cells to be discarded are produced in the production of the enzyme, so that these methods are not industrially desirable due to the defect that not only the amount of the bacterial cells to be discarded is increased in proportion to the increasing amount of the enzyme, but also the load on the separating operations of the bacterial cells and the salts after the enzyme reaction is increased.

In the hydrolysis reaction of amino acid amides, optically active amino acids and ammonia as a by-product are produced. In this connection, it has been described, for example, in Japanese Patent No. 4139773 and WO 2003/020929 that ammonium buffer solutions have been used as the buffer solution for the enzyme reaction of the enzyme. Furthermore, it has been described in Japanese Patent No. 3647065 that aqueous ammonia has been added for adjusting pH of an enzymatic reaction with an enzyme for hydrolyzing an amino acid amide. Thus, it may be predicated in the enzymatic reaction of an enzyme capable of hydrolyzing the amino acid amide that the negligible or no influence of ammonia or an ammonium ion is observed in the enzymatic reaction, since the use of an ammonia type buffer solution or the addition of ammonia for adjusting pH is generally conducted in this enzymatic reaction.

Japanese Patent Laid-Open Publication No. 2006-180751 discloses a method for removing ammonia as a by-product during the hydrolysis reaction with an enzyme capable of an amino acid amide. However, this method comprises the operation carried out at a high temperature for improving the solubility of an amino acid produced by the hydrolysis reaction to prevent the crystallization of the amino acid and thus is not a technique for improving the productivity by removing ammonia. Moreover, while it has been described in Japanese Patent Laid-Open Publication No. 2006-180751 that ammonia is not desirable to a microbial catalyst having amide hydrolyzing activity from the viewpoint of the activity and the stability of the catalyst, no measure to counter ammonia has not been described. It can rather be said that the use of an ammonia type buffer solution or the addition of ammonia for adjusting pH is generally conducted in this enzymatic reaction for hydrolyzing the amino acid amides.

For example, Japanese Patent Laid-Open Publication No. 2006-340630 discloses that ammonia affects the activity of an enzyme capable of amino acid amides and provides a countermeasure for avoid the influence of ammonia by searching new enzymes, which requires a great deal of labor and time and does not always find a new enzyme which is hardly affected by ammonia. In addition, even if the enzyme can avoid the influence of ammonia on a particular substrate, it may be influenced by ammonia on the other substrates. Thus, the search of a new enzyme cannot be said an effective means.

There has been described a method for preparing an optically active amino acid by the hydrolysis reaction with an enzyme capable of stereoselectively hydrolyzing an amino acid amide, after which reaction ammonia or an ammonium salt is removed by the addition of a base or the stripping, for example, in Japanese Patent Laid-Open Publication No. 2007-254439. However, this method aims at removing the ammonium salt having a low solubility in an organic solvent and then ammonia after the hydrolysis reaction.

It has been described in Japanese Patent Laid-Open Publication No. 2004-521623 and Japanese Patent Laid-Open Publication No. 61-285996 that ammonia can be removed by the distillation or the addition of salts as the conventional methods for removing ammonia in the hydrolysis reaction with the other enzymes. However, there have been described no methods as the combination of the reaction with a microbial catalyst having the amide hydrolysis activity and these methods for removing ammonia.

### SUMMARY OF THE INVENTION

The present inventors have examined the enzymatic reaction for stereoselectively hydrolyzing DL-tert-leucine amide, and as a result, raised a problem that when the concentration of the amino acid amide as the raw material was increased without the increase of the amounts of neither an acid for adjusting pH nor of an enzyme, the reaction was not completed due to the deactivation of the enzyme along with the progress of the reaction in spite of the pH of the enzyme in the optimal range to lead to the stagnation of the reaction. Furthermore, a problem was also raised even in the use of an immobilized biocatalyst such as an immobilized enzyme, that the activity of the immobilized biocatalyst was strikingly lowered to the almost inability to the repeated use of the immobilized biocatalyst. Thus, a method for adding a raw material in portions, a method for sequentially taking out an amino acid as a product and a method for using these methods integrally were further examined, but the problem remained unsolved.

Thus, the present inventors have earnestly studied the inhibitory factors of an enzyme capable of hydrolyzing stereoselectively DL-tert-leucine amide and extraordinarily found that the enzyme used in the present invention is inhibited by ammonia and an ammonium ion in the presence of DL-tert-leucine amide as a substrate, although the information that the enzyme is not inhibited by ammonia in the presence of the other substrate (DL-2-methylcysteine amide hydrochloride) has been obtained (Referential Example 3). Now, the present inventors have found that the lowering of the enzymatic activity can be substantially reduced by decreasing the sum of the concentrations of ammonia and an ammonium ion by continuously or intermittently separating ammonia from the reaction solution during the hydrolysis reaction with the enzyme and an immobilized biocatalyst, if used, can be used repeatedly, and have successfully found the solution to the problem of the decreased reaction rate along the decrease of the activity. In this connection, it has been also found that the evaporation under reduced pressure and the adsorption on a cation exchange resin or zeolite are effective for the separation of ammonia from the reaction solution. Thus, the productivity per reactor could have been improved by increasing the concentration of the amino acid amide as the raw material in the reaction solution. It has been found as a result thereof that the productivity may be improved substantially without increasing a waste salt or waste cells as the waste matters of the reaction and that an optically active tert-leucine or an optically active tert-leucine amide can be prepared in high quality and inexpensively. The present invention is based on the information described above.

Thus, the object of the present invention is to provide a method for preparing optically active tert-leucine or optically active tert-leucine amide, in which the concentration of DL-tert-leucine amide as a raw material in the reaction solution can be enhanced without the need of increasing the amount of the cells or a pH-adjusting acid by maintaining the activity per unit amount of the enzyme, the cells or the cell treatment product and per unit time period at a high level in the enzymatic reaction for hydrolyzing the DL-tert-leucine amide stereoselectively, and consequently the productivity of optically active tert-leucine or optically active tert-leucine amide can be improved to a great extent without increasing the generation of a waste salt or waste cells.

The present invention relates to a method for producing D- or L-tert-leucine or D- or L-tert-leucine amide by reacting DL-tert-leucine amide with a biocatalyst selected from the group consisting of an enzyme capable of hydrolyzing the DL-tert-leucine amide stereoselectively, cells of a microorganism having the enzyme, a material produced by the treatment of the cells, an immobilized enzyme produced by immobilizing the enzyme onto a carrier, immobilized cells produced by immobilizing the cells onto a carrier, and an immobilized cell treatment product obtained by immobilizing the cell treatment product onto a carrier to thereby hydrolyze the DL-tert-leucine amide, wherein the hydrolysis is carried out with separating ammonia produced by the hydrolysis from the hydrolysis reaction solution.

According to the preferred embodiment of the present invention, in the method of the present invention, ammonia is separated from the reaction solution by
placing the reaction solution under reduced pressure to evaporate the ammonia, or
adsorbing the ammonia in the reaction solution onto a cation exchange resin or zeolite.

According to one preferred embodiment of the present invention, in the method of the present invention, ammonia is separated from the reaction solution by placing the reaction solution under reduced pressure to evaporate the ammonia.

According to another preferred embodiment of the present invention, in the method of the present invention, ammonia is separated from the reaction solution by adsorbing the ammonia in the reaction solution onto a cation exchange resin.

According to the still another preferred embodiment of the present invention, in the method of the present invention, ammonia is separated from the reaction solution by adsorbing the ammonia in the reaction solution onto zeolite.

According to one preferred embodiment of the present invention, in the method of the present invention, the enzyme is the one derived from Xanthobacter flavus.

According to one preferred embodiment of the present invention, in the method of the present invention, the microorganism is the one derived from Xanthobacter flavus and having the gene of an enzyme capable of hydrolyzing the DL-tert-leucine amide stereoselectively introduced therein.

According to one more preferred embodiment of the present invention, in the method of the present invention, the microorganism is pMCA1/JM109 (FERM BP-10334).

According to another preferred embodiment of the present invention, in the method of the present invention, the sum of the concentrations of ammonia and an ammonium ion in the reaction solution is in the range of 7000 ppm or less.

Furthermore, according to another embodiment of the present invention, a method for preparing a D- or L-amino acid or a D- or L-amino acid amide by reacting a DL-amino acid amide with an enzyme capable of hydrolyzing the DL-amino acid amide stereoselectively or a microorganism containing the enzyme or a microorganism treatment product to hydrolyze the amide, characterized in that the hydrolysis is carried out with separating ammonia produced by the hydrolysis from the reaction solution.

According to the present invention, in the enzymatic reaction stereoselectively hydrolyzing the DL-tert-leucine amide, the raw material concentration can be increased without increasing a waste salt or waste cells, so that D- or L-tert-leucine or D- or L-tert-leucine amide, particularly L-tert-leucine or D-tert-leucine amide can be prepared, in which the load of concentration operation and the like in the later steps can be reduced and the productivity per reactor has been substantially improved.

### BRIEF DESCRIPTION OF THE DRAWINGS

Figure 1 shows the result of Example A.
Figure 2 shows the schematic diagram of an apparatus used in Examples A-3 and D-2.
Figure 3 shows the result of Example B.
Figure 4 shows the result of Example C.
Figure 5 shows the result of Example D.
Figure 6 shows the result of Example E.
Figure 7 shows the result of Example F.

### DETAILED DESCRIPTION OF THE INVENTION

The present invention is now described with reference to the embodiments. The scope of the present invention is not limited by the embodiments and examples.

In the present invention, the term "DL-tert-leucine amide" means the mixture or racemic isomer of D- or L-tert-leucine amide.

In the present invention, a biocatalyst selected from the group consisting of an enzyme having an activity of stereoselectively hydrolyzing L- or D-tert-leucine amide corresponding to L- or D-tert-leucine, cells of a microorganism having the enzyme, a material produced by the treatment of the cells, an immobilized enzyme produced by immobilizing the enzyme onto a carrier, immobilized cells produced by immobilizing the cells onto a carrier, and an immobilized cell treatment product obtained by immobilizing the cell treatment product onto a carrier is used in the stereoselective hydrolysis of DL-tert-leucine amide.

Microorganisms having an enzyme capable of hydrolyzing stereoselectively DL-tert-leucine amide include, for example, those belonging to Xanthobacter, Protaminobacter, Mycobacterium, and Mycoplana genera. Specifically, there are mentioned Xanthobacter flavus, Protaminobacter alboflavus, Mycobacterium methanolica, Mycobacterium methanolica, Mycoplana ramosa, Mycoplana dimorpha, Variovorax paradoxus, and the like, but not limited thereto. More specific examples include Xanthobacter flavus NCIB 10071T, Protaminobacter alboflavus ATCC 8458, Mycobacterium methanolica BT-84 (FERM P8823), Mycobacterium methanolica P-23 (FERM P8825), Mycoplana ramosa NCIB 9440T, Mycoplana dimorpha ATCC 4279T, and Variovorax paradoxus DSM 14468.

Moreover, variants derived from these microorganisms by artificial mutational means or recombinant strains derived from these microorganisms by genetic methods such as cell fusion or genetic recombination including, for example, the strain pMCA1/JM109 (FERM BP-10334) having an enzyme capable of hydrolyzing stereoselectively an amino acid amide derived from Xanthobacter flavus by introducing the gene of the enzyme may also be used in the present invention. In addition, the cell treatment product includes, for example, a concentrated cell solution, dry cells, cell debris, a cell extract or a purified enzyme. Furthermore, an enzyme obtained by a method using no microorganisms such as a cell-free protein synthesis system may also be used.

The immobilized biocatalyst that the enzyme or the cells or the cell treatment product of a microorganism having the enzyme have been immobilized on a carrier (i.e., immobilized enzyme, immobilized cells, and immobilized cell treatment product) can be prepared by the methods well known in the art such as crosslinking method, covalent binding method, physical adsorption method and entrapment method. The carriers used in the immobilization include the conventional ones which may be appropriately used depending on the immobilization methods, specifically the carriers for the immobilization with natural polymers such as alginic acid, collagen, gelatin, agar, κ-carrageenan and the like, synthetic polymers polyacrylamide, photo-curable resin, urethane polymer and the like, and microcapsules.

DL-tert-leucine amide as the raw material and the substrate of the enzymatic reaction is represented by the following general formula (1), in which R represents a tert-butyl group:

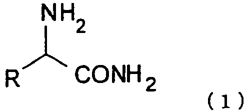

To the aqueous solution of the DL-tert-leucine amide described above were added the enzyme, the cells of a microorganism having the enzyme, the cell treatment product, the immobilized enzyme, the immobilized cells or the immobilized cell treatment product and the other ingredients, if necessary, to prepare a reaction solution, which is subjected to stereoselective hydrolysis simultaneously with the separation of ammonia produced to prepare the optically active tert-leucine represented by the following general formula (2) or the optically active tert-leucine amide as the enantiomer thereof and represented by the following general formula (3). In this connection, the group R in the general formulae (2) and (3) refers to the same group R in the general formula (1).

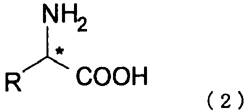

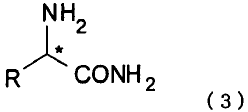

The concentration of DL-tert-leucine amide as the substrate in the reaction solution before stereoselective hydrolysis is preferably in the range from 0.01% by weight to the saturated concentration of DL-tert-leucine amide, more preferably 10 to 30% by weight. By setting the concentration of DL-tert-leucine amide within the range described above, the productivity per volume of the reaction solution can be enhanced since the substrate concentration will not be decreased excessively and the deactivation of the enzyme due to the high substrate concentration can be avoided.

The pH range suitable for the stereoselective hydrolysis of the DL-amino acid amide varies depending on enzymes to be used and cannot be indiscriminately defined. However, when pMCA1/JM109 (FERM BP-10334) is used as the enzyme, the reaction proceeds suitably at pH 6 to 10. The aqueous solution of the DL-tert-leucine amide has a pH at around 10.5, and thus it is necessary to adjust the pH by adding an acid to the aqueous solution for conducting the stereoselective hydrolysis under the optimal pH condition.

The acid to be added for adjusting pH is not specifically limited and may be a mineral acid or an organic acid, among which hydrochloric acid and acetic acid are suitably used. The amount of the acid to be used may be determined so as the pH to be in the range described above. For instance, hydrochloric acid is used in an amount of 0.005 to 0.5 molar times, preferably 0.05 to 0.4 molar times to DL-tert-leucine amide, and acetic acid is used in an amount of 0.005 to 1 molar times, preferably 0.05 to 0.7 molar times to DL-tert-leucine amide.

Some enzymes may improve the rate of stereoselective hydrolysis by the addition of a metal ion, and in such cases a variety of metal ions such as Ca²⁺, Co²⁺, Cu²⁺, Fe³⁺, Mg²⁺, Mn²⁺, Ni²⁺, Zn²⁺ and the like may be added to the reaction solution, for example, in an amount of 1 to 100 ppm.

In the stereoselective hydrolysis reaction of DL-tert-leucine amide with an enzyme capable of hydrolyzing stereoselectively an amino acid amide, ammonia is generated in equimolar amount to the L- or D-tert-leucine to be produced. This ammonia is separated continuously or intermittently during the hydrolysis reaction from the reaction solution. The enzyme to be used in the present invention is inhibited by ammonia in the case of using DL-tert-leucine amide as a substrate, but the lowering of the reaction rate can be suppressed by separating ammonia from reaction solution during the hydrolysis to prevent the deactivation of the enzyme due to the increase of ammonia and in its turn the concentration of the raw material can be increased.

The deactivation of the enzyme due to ammonia and an ammonium ion is not only caused by the sum of the concentrations of ammonia and ammonium ion, but depends on enzymes and temperatures. Therefore, the concentration of ammonia in the reaction solution cannot be defined unconditionally. When the stereoselective hydrolysis reaction of DL-tert-leucine amide is carried out with pMCAl/JM109 (FERM BP-10334) as the enzyme in an aqueous solution at 40°C, the condition of separating ammonia is set so as the sum of the concentrations of ammonia concentration and an ammonium ion in the reaction solution to be in the range of 7000 ppm or less, preferably 6000 ppm or less.

When the enzyme, the microorganism having the enzyme or the treatment product thereof is recovered by the method well known to a person skillful in the art such as ultrafiltration after the reaction, the condition of separating ammonia is set so as the sum of the concentrations of ammonia concentration and an ammonium ion in the reaction solution to be preferably in the range of 3500 ppm or less, more preferably 2500 ppm or less.

When the enzyme capable of hydrolyzing stereoselectively the amino acid amide or the cell treatment product containing the enzyme is used as an immobilized biocatalyst, the deactivation of the enzyme by ammonia and the ammonium ion varies depending on the factors such as enzymes, immobilization methods and temperatures. For instance, when stereoselective hydrolysis is conducted with the cell pMCA1/JM109 (FERM BP-10334) as the immobilized biocatalyst in order to reuse the immobilized biocatalyst, the condition of separating ammonia is set so as the sum of the concentrations of ammonia concentration and an ammonium ion in the reaction solution to be in the range of 3500 ppm or less, preferably 2500 ppm or less.

Ammonia may be separated from either place of a reactor in which hydrolysis reaction is conducted or an ammonia separating apparatus provided apart from the reactor.

As the method for separating ammonia from the reaction solution, the reduced pressure method in which the reaction solution is placed under reduced pressure, the method in which ammonia is adsorbed on a cation exchange resin or the method in which ammonia is adsorbed on zeolite can be used.

The other methods for separating ammonia from the reaction solution include the method for aerating the reaction solution and the method for heating the reaction solution. However, the method for ventilating the reaction solution may unfavorably lead to incomplete reaction due to low amount of ammonia separable from the reaction solution. Furthermore, the method for heating the reaction solution may also unfavorably lead to the deactivation of the enzyme at a temperature for separating a sufficient amount of ammonia.

### Reduced pressure method

In order to separate ammonia from the reaction solution, the reaction solution can be placed under reduced pressure to evaporate the ammonia.

In the reaction by the reduced pressure method, when the cell pMCAl/JM109 (FERM BP-10334) is used as the enzyme and the temperature of the reaction solution is set at 40°C, the pressure during the hydrolysis reaction of the DL-tert-leucine amide is preferably controlled in the range of not less than 40 mmHg which is the boiling pressure of the reaction solution to not more than 90 mmHg which is 50 mmHg higher than the boiling pressure.

If the amount of the reaction solution is decreased by the evaporation of water along with the separation of ammonia, water may be added externally.

### Adsorption method by a cation exchange resin

In order to separate ammonia from the reaction solution, ammonia in the reaction solution can be adsorbed on a cation exchange resin.

When a cation exchange resin is used, the cation exchange resin to be added to the reaction solution may be either a strong acidic ion exchange resin or a weak acidic ion exchange resin capable of adsorbing ammonia. Moreover, the resin to be selected is not limited by its types, forms or the like, but it may be appropriately determined by taking account of its adsorption capacity, ion exchange capacity, strength, price and the like, and for example, resins such as DOWEX 50WX8 (Dow Chemical) DIAION SK110 (Mitsubishi Chemical) and AMBERITE IR120B (Rohm and Haas) may be preferably used.

The amount of the cation exchange resin to be added to the reaction solution varies depending on the ion exchange capacity and adsorption capacity of the resin, but it needs only to be an amount satisfactory for adsorbing ammonia produced in the hydrolysis reaction. If the amount of the cation exchange resin to be added to the reaction solution is less than that sufficient for adsorbing ammonia produced in the hydrolysis reaction, the coexisting effect of the cation exchange resin cannot be satisfactorily anticipated, and the aimed reaction may not be completed due to the lowering of the reaction rate with the progress of the reaction. Moreover, when the cation exchange resin to be added to the reaction solution is in an excessively large amount, the amino acid, the amino acid amide, the cells and the enzyme may be adsorbed on the cation exchange resin resulting in the decrease of recovery or the rate of reaction. Thus, the suitable cation exchange resin is preferably added to the reaction solution in such amount that the ion exchange capacity of the resin is 0.05 to 2 times the amount of ammonia to be produced.

The cation exchange resin to be used needs only to be of an active type, and the used cation exchange resin can be used again by the activation of the resin.

The method for adding the cation exchange resin to the reaction solution needs only to substantially contact the cation exchange resin with the reaction solution, and for example, any method such as a method for adding directly a cation exchange resin to a reaction solution in a stirring reaction vessel for use of the resin in a suspension form, a method for filling a filter cloth or membrane or a cage type vessel with a cation exchange resin to place the resin in a reaction vessel, and a method for flowing or circulating a reaction solution through a tower which is filled with a cation exchange resin can be used. The method for adding directly a cation exchange resin to a reaction solution in a stirring reaction vessel for use of the resin in a suspension form is a simple and convenient method, but the method for filling a filter cloth or membrane or a cage type vessel with a cation exchange resin to place the resin in a reaction vessel or the method for flowing or circulating a reaction solution through a tower which is filled with a cation exchange resin is rather preferably used in consideration of the damage of the resin due to stirring and the laborious recovery of the resin after reaction. It is also possible to use a cation exchange resin formed into membrane or fiber.

### Adsorption method with zeolite

In order to separate ammonia from the reaction solution, ammonia can be adsorbed onto zeolite in the reaction solution.

When zeolite is used, zeolite to be added to the reaction solution needs only to have an ability of adsorbing ammonia during the hydrolysis reaction, and any type of zeolite can be used without limitation including, for example, Mordenite, Zeolite Y, Chabazite and the like, which may be used taking in comprehensive consideration of adsorption capacity, ion exchange capacity, surface area per unit weight, strength, price and the like.

Zeolite to be added to the reaction solution is preferably used in a compact form which has a high strength and is hard to powder rather than in a powder form. The powdered zeolite adsorbs also the enzyme in addition to ammonia thus inhibiting the reaction. The zeolite compact having a low strength tends to be partly powdered thus inhibiting the reaction. In order to avoid the adsorption of the enzyme onto the powdered zeolite and the inhibition of the reaction, the method for filling a filter cloth or membrane or a cage type vessel with the compact to place it in a reaction vessel or the method for flowing or circulating a reaction solution through a tower which is filled with the compact is preferably used.

The amount of the zeolite to be added to the reaction solution varies depending on the types, adsorption capacity, ion exchange capacity and surface area per unit weight of zeolite, but it needs only to be an amount satisfactory for adsorbing ammonia produced in the hydrolysis reaction. If the amount of the zeolite to be added to the reaction solution is less than that sufficient for adsorbing ammonia produced in the hydrolysis reaction, the coexisting effect of the zeolite in the reaction solution will not be satisfactorily exhibited, and the aimed reaction may not be completed due to the lowering of the reaction rate with the progress of the reaction. Moreover, when the zeolite to be added to the reaction solution is in an excessively large amount, the amino acid, the amino acid amide, the cells and the enzyme may be adsorbed on the zeolite resulting in the decrease of recovery or the rate of reaction. Thus, the suitable zeolite is preferably added to the reaction solution in such amount that the ion exchange capacity of the zeolite is 0.05 to 2 times the amount of ammonia to be produced.

The zeolite to be used needs only to be in a situation capable of adsorbing ammonia and includes the proton type, and the used zeolite can be used again by treating it so as to be capable of adsorbing ammonia.

The method for adding the zeolite to the reaction solution needs only to substantially contact the zeolite with the reaction solution, and for example, any method such as a method for adding directly the zeolite to a reaction solution in a stirring reaction vessel for use of the zeolite in a suspension form, a method for filling a filter cloth or membrane or a cage type vessel with the zeolite to place the zeolite in a reaction vessel, and a method for flowing or circulating a reaction solution through a tower which is filled with the zeolite can be used. While the method for adding directly the zeolite to a reaction solution in a stirring reaction vessel for use of the zeolite in a suspension form is a simple and convenient method, it has the problems of the powdering of the compact due to stirring, the recovery of the zeolite after reaction and the adsorption of the cells on the zeolite. Thus, the method for filling a filter cloth or membrane or a cage type vessel with the zeolite to place the zeolite in a reaction vessel can exhibit the most preferable effect.

After the enzymatic reaction, optically active tert-leucine or optically active tert-leucine amide can be obtained from the reaction solution by the method well known to a person skillful in the art. For instance, the cells, proteins and nucleic acids are removed by adsorbing these materials on active carbon from the reaction solution after the enzymatic reaction, and optically active tert-leucine can be crystallized by taking advantage of the difference of solubilities between the optically active tert-leucine and the optically active tert-leucine amide as the enantiomer thereof by adding, for example, 2-methyl-1-propanol to the reaction solution. Thus, the optically active tert-leucine can be obtained as the crystalline form by filtering the solution. Furthermore, the filtrate obtained can be concentrated to dryness to give the optically active tert-leucine amide.

### EXAMPLES

The present invention is now described in detail by way of examples, but not limited thereby.

### Experimental method and measurement

The progress of reaction and the optical purity were measured by high performance liquid chromatography (HPLC), and the sum of the ammonia concentration and the ammonium ion concentration was measured by capillary electrophoresis. Analytical conditions are described below.

### [HPLC analytical condition 1]

Column: Lichrosorb RP-18 (4.6ϕ×250mm)
Eluent: 50 mM aqueous solution of perchloric acid
Flow rate: 0.5 ml/min
Detection: RI

### [HPLC analytical condition 2]

Column: Sumichiral OA-5000 (4.6ϕ×50mm)
Eluent: 10 mM aqueous solution of copper sulfate
Flow rate: 0.5 ml/min
Detection: UV 254nm

### [HPLC analytical condition 3]

Column: LiChrosorb 100RP-18 (4.6ϕ×250mm)
Column temperature: 40°C
Eluent: 50 mM aqueous solution of perchloric acid
Flow rate: 0.5 ml/min
Detection: RI

### [Analytical condition of ammonia]

Apparatus: Capillary electrophoresis system 3DCE (Agilent)
Capillary length: 40 cm

### Referential Example 1: Preparation of cells

The recombinant strain pMCA1/JM109 (FERM BP-10334) having the L-tert-leucine amide stereoselective hydrolysis enzyme to be used in the following Examples and Comparative Examples was cultured in Turbo medium (Athena Environmental Sciences, Inc.; purchased from Funakoshi Corp.) at 37°C and centrifuged to give a cell concentrate solution (containing 6.7% by weight of dry cells).

In this connection, the recombinant strain pMCA1/JM109 of Escherichia coli has been deposited to International Patent Organism Depositary, National Institute of Advanced Industrial Science and Technology on May 21, 2004 (Heisei 16) (original deposit date) (Tsukuba Central 6, 1-1, Higashi, Tsukuba, Ibaraki 305-8566, Japan) with accession no. FERM BP-10334.

### Referential Example 2: Preparation of immobilized biocatalyst

An immobilized biocatalyst was prepared in the following manner. 27.2 g of PEG-1000 dimethacrylate (Shi-Nakamura Chemicaol Co., Ltd), 0.9 g of N,N'-methylenebisacrylamide, 1.0 g of tetramethylethylenediamine, 0.03 g of ammonium peroxodisulfate, 52.2 g of the concentrated cell solution obtained in Referential Example 1, and 18.7 g of pure water were mixed homogeneously and solidified by leaving to stand at ambient temperature. After solidified, the solid product was cut into a size of ca. 3 mm and used as an immobilized biocatalyst in the following Examples.

### Referential Example 3: Effect of ammonia on the other substrate in the presence of recombinant pMCA1/JM109

To a 500 ml flask was added 16.3 g of DL-2-methylcysteine amide hydrochloride (0.096 mole), which was dissolved in 141.3 g of water. To the aqueous solution was added 3.2 g of 28% aqueous ammonia, and the mixture was stirred. In this time, ammonia concentration was 5500 ppm. In addition, 2.97 mg of manganese chloride tetrahydrate and then 0.09 g of the concentrated cell solution of the recombinant pMCA1/JM109 prepared in Referential Example 1 were added, and the mixture was stirred at 30°C under nitrogen stream for stereoselective hydrolysis. The examination of the rate of reaction with the HPLC condition 3 revealed that at least 95% of L-2-methylcysteine amide was converted into L-2-methylcysteine after 24 hours of the reaction and the sum of the ammonia concentration and the ammonium ion concentration in the reaction was 10000 ppm.

### Example A: Reduced pressure/enzymatic reaction

### Example A-1: Reduced pressure (40 mmHg)/enzymatic reaction

To a 200 ml flask was added 20.0 g of DL-tert-leucine amide (0.15 mole), which was dissolved in 78.4 g of water. To the aqueous solution was added 1.86 g of acetic acid (0.031 mole), and the mixture was stirred. At this time, the pH of the mixture was 8.3. In addition, 6.88 mg of manganese chloride tetrahydrate and then 0.60 g of the concentrated cell solution of the recombinant strain pMCA1/JM109 were added for stereoselective hydrolysis with stirring at 40°C under reduced pressure of 40 mmHg.

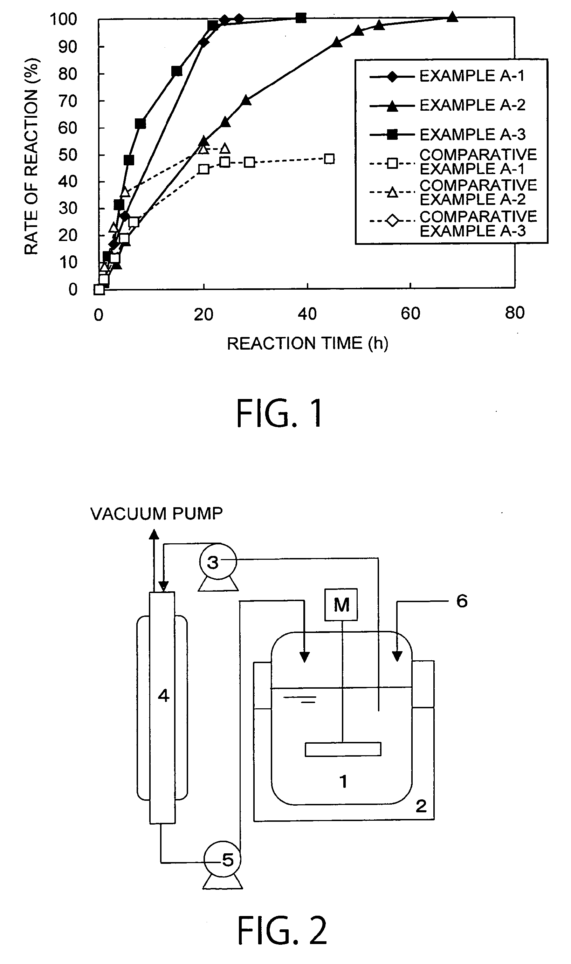

The time-dependent change of the rate of reaction measured with the HPLC analytical condition 1 is illustrated in Figure 1. The rate of reaction in Figure 1 refers to the percentage of L-tert-leucine amide converted into L-tert-leucine in the raw material DL-tert-leucine amide. After 27 hours of reaction, at least 99.5% of L-tert-leucine amide was converted into L-tert-leucine. Moreover, the measurement after 27 hours of reaction with the HPLC analytical condition 2 revealed that L-tert-leucine had an optical purity of 100% ee. The sum of the ammonia concentration and the ammonium ion concentration reached 5000 ppm after 5 hours of reaction and was always maintained at this level or less by removing ammonia from the reaction system.

A filtrate from which the cells had been removed by filtering the reaction solution having active carbon and a filter aid added thereto was obtained. The mixture of the filtrate with 65 g of 2-methyl-1-propanol was subjected to azeotropic dehydration until the hydrated concentration reached 1% under 20 kPa for solvent displacement operation. The azeotropic dehydration proceeded at a boiling point of 60°C, and the boiling point reached 76°C at the completion of azeotropic dehydration. L-tert-leucine crystallized by solvent displacement was collected by suction filtration, washed with 30 g of 2-methyl-1-propanol warmed to 70°C and further with 90 g of acetone at 25°C, and subjected to vacuum drying at ambient temperature to give 9.8 g of white powder. Analysis of the L-tert-leucine according to the HPLC condition 1 revealed that the product had a chemical purity of 99.8%. The optical purity of the product measured according to the HPLC condition 2 was 99.5% or more.

### Example A-2: Reduced pressure (65 mmHg)/enzymatic reaction

The reaction was conducted in the same manner as that in Example A-1 except that the stereoselective hydrolysis was conducted at 65 mmHg. The result of the reaction is illustrated in Figure 1. After 68 hours of reaction, at least 99.5% of L-tert-leucine amide was converted into L-tert-leucine. Moreover, L-tert-leucine had an optical purity of 100% ee. The sum of the ammonia concentration and the ammonium ion concentration reached 6000 ppm after 7 hours of reaction and was always maintained at this level or less by removing ammonia from the reaction system.

### Example A-3: Reduced pressure (40 mmHg)/enzymatic reaction (provided with an ammonia separating apparatus)

### 1) Experimental apparatus

Apparatus is schematically illustrated in Figure 2.
The reaction solution added to the reaction vessel 1 is reacted with stirring under atmospheric air while controlling the temperature of the reaction solution with the heater 2. At the same time, a portion of the reaction solution is fed into the evaporator 4 as an ammonia separating apparatus through the transfer pump of the reaction solution 3. The evaporator 4 is maintained under reduced pressure by the vacuum pump and also temperature controlled, and the reaction solution after separating ammonia is sent back to the reaction vessel 1 through the transfer pump of the reaction solution 5. Moreover, water can be added to the reaction vessel 1 through the water supply line 6.

### 2) Stereoselective hydrolysis

To a 500 ml flask as a reaction vessel was added 50.39 g of DL-tert-leucine amide (0.38 mole), which was dissolved in 194.24 g of water. To the aqueous solution was added 4.62 g of acetic acid (0.031 mole), and the mixture was stirred. At this time, the pH of the mixture was 8.3. In addition, 9.01 mg of manganese chloride tetrahydrate and then 1.82 g of the concentrated cell solution of the recombinant strain pMCA1/JM109 were added for stereoselective hydrolysis with stirring at 40°C under reduced pressure of 40 mmHg. Just after the initiation of the reaction, a portion of the reaction solution was continuously circulated through the evaporator at 40 mmHg for removing ammonia produced with the enzymatic reaction. Water evaporated from the evaporator was supplied from the water supply line. The result of the reaction is illustrated in Figure 1. After 39 hours of reaction, at least 99.5% of L-tert-leucine amide was converted into L-tert-leucine. The sum of the ammonia concentration and the ammonium ion concentration reached 3500 ppm after 6 hours of reaction and was always maintained at this level or less by removing ammonia from the reaction system.

### Comparative Example A-1

The reaction was conducted in the same manner as that in Example A-1 except that the stereoselective hydrolysis was conducted at atmospheric pressure. The result of the reaction is illustrated in Figure 1. After 45 hours of reaction, 47.4% of L-tert-leucine amide was converted into L-tert-leucine and the rest remained as L-tert-leucine amide. The sum of the ammonia concentration and the ammonium ion concentration increased with the advance of the reaction until 45 hours of reaction up to the maximum of 7800 ppm. Thus, the enzymatic reaction was not completed under atmospheric pressure condition.

### Comparative Example A-2

The reaction was conducted in the same manner as that in Example A-1 except that the stereoselective hydrolysis was conducted at atmospheric pressure and nitrogen was circulated at the rate of 100 ml/min from the bottom part of the reaction solution. The result of the reaction is illustrated in Figure 1. After 24 hours of reaction, 52.0% of L-tert-leucine amide was converted into L-tert-leucine and the rest remained as L-tert-leucine amide. The sum of the ammonia concentration and the ammonium ion concentration increased with the advance of the reaction until 24 hours of reaction up to the maximum of 7200 ppm. Thus, the enzymatic reaction was not completed under atmospheric pressure condition.

### Comparative Example A-3

The reaction was conducted in the same manner as that in Example A-1 except that the stereoselective hydrolysis was conducted at atmospheric pressure and at a temperature of 55°C. The result of the reaction is illustrated in Figure 1. After 24 hours of reaction, 15.8% of L-tert-leucine amide was converted into L-tert-leucine and the rest remained as L-tert-leucine amide. The sum of the ammonia concentration and the ammonium ion concentration increased with the advance of the reaction until 24 hours of reaction up to the maximum of 2000 ppm. Thus, the enzymatic reaction with use of the combination of the enzyme and the substrate was not completed due to the deactivation of the activation at high temperature.

### Example B: Absorption of cation exchange resin/enzymatic reaction

### Example B-1

To a 100 ml flask was added 12.13 g of DL-tert-leucine amide (0.093 mole), which was dissolved in 44.31 g of water. To the aqueous solution was added 3.3 g of acetic acid (0.056 mole), and the mixture was stirred. At this time, the pH of the mixture was 8.3. In addition, 5.97 mg of manganese chloride tetrahydrate was added to the mixture to prepare the stock solution of the reaction. A 10.0 g portion of the stock solution was placed in a test tube, and 0.12 g of the concentrated culture solution of the recombinant strainpMCA1/)M109 (containing 0.008 g by weight of dry cells) was inoculated for stereoselective hydrolysis with stirring at 40°C. The cation exchange resin DOWEX 50WX8 (Dow Chemical) in an amount of 2.5 g was suspended into the reaction solution.

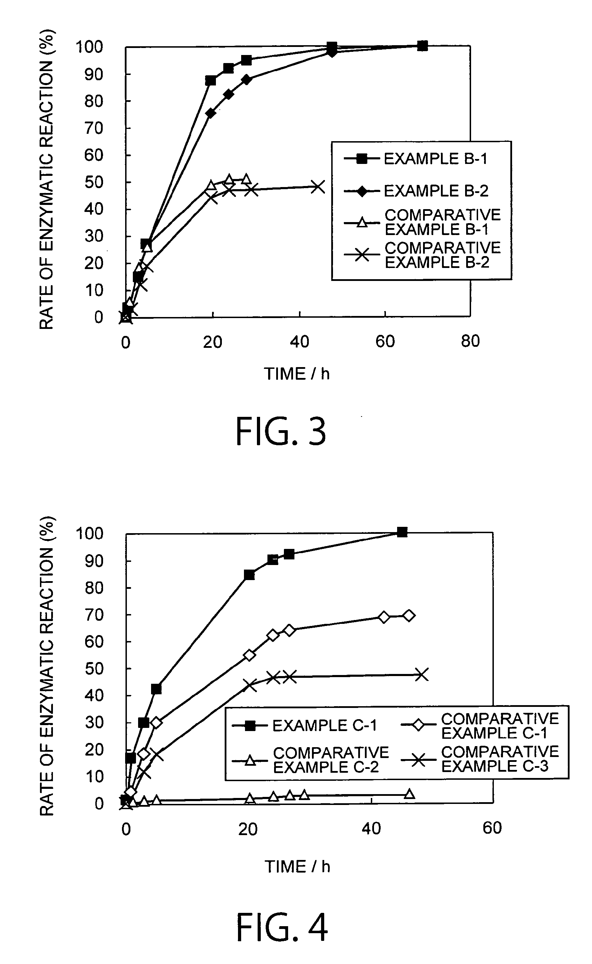

The progress of the reaction was confirmed by the high performance liquid chromatography (HPLC) condition 1. The result of the reaction is illustrated in Figure 3. After 69 hours of reaction, 99.8% of L-tert-leucine amide was converted into L-tert-leucine. The sum of the ammonia concentration and the ammonium ion concentration reached 5500 ppm after 20 hours of reaction and was always maintained at this level or less by removing ammonia from the reaction system.

A filtrate from which the cells and the cation exchange resin had been removed by filtering the reaction solution having active carbon and a filter aid added thereto was obtained. The mixture of the filtrate with 35 g of 2-methyl-1-propanol was subjected to azeotropic dehydration until the hydrated concentration reached 1% under 20 kPa for solvent displacement operation. The azeotropic dehydration proceeded at a boiling point of 60°C, and the boiling point reached 76°C at the completion of azeotropic dehydration. L-tert-leucine crystallized by solvent displacement was collected by suction filtration, washed with 25 g of 2-methyl-1-propanol warmed to 70°C and further with 75 g of acetone at 25°C, and subjected to vacuum drying at ambient temperature to give 5.9 g of white powder. Analysis of the L-tert-leucine according to the HPLC condition 1 revealed that the product had a chemical purity of 99.5%. The optical purity of the product measured according to the HPLC condition 2 was 99.5% or more.

### Example B-2

Under the same condition as in Example B-1, a bag of 2.5 g of the cation exchange resin DOWEX 50WX8 (Dow Chemical) packed in unwoven fabric was added to the reaction solution. The result of the enzymatic reaction is illustrated in Figure 3. After 45 hours of reaction, 99.8% of L-tert-leucine amide was converted into L-tert-leucine. The sum of the ammonia concentration and the ammonium ion concentration reached 6000 ppm after 20 hours of reaction and was always maintained at this level or less by removing ammonia from the reaction system.

### Comparative Example B-1

Under the same condition as in Example B-1, the reaction was conducted in the absence of a cation exchange resin. The result of the reaction is illustrated in Figure 3. After 46 hours of reaction, 68.5% of L-tert-leucine amide was converted into L-tert-leucine, and the rest remained as L-tert-leucine amide. The sum of the ammonia concentration and ammonium ion concentration in the reaction solution was increased with the progress of the reaction until 46 hours and reached the maximum of 8000 ppm.

### Comparative Example B-2

To a 200 ml flask was added 20.0 g of DL-tert-leucine amide (0.15 mole), which was dissolved in 78.4 g of water. To the aqueous solution was added 1.86 g of acetic acid (0.031 mole), and the mixture was stirred. At this time, the pH of the mixture was 8.3. In addition, 6.88 mg of manganese chloride tetrahydrate and then 0.60 g of the concentrated cell solution obtained in Referential Example 1 were added and the mixture was subjected to stereoselective hydrolysis with stirring at 40°C in the absence of a cation exchange resin. The result of the reaction is illustrated in Figure 3. After 45 hours of reaction, 47.4% of L-tert-leucine amide was converted into L-tert-leucine and the rest remained as L-tert-leucine amide. The sum of the ammonia concentration and the ammonium ion concentration was increased with the progress of the reaction until 45 hours and reached the maximum of 7800 ppm.

### Example C: Adsorption of zeolite/enzymatic reaction

### Example C-1

To a 100 ml flask was added 12.13 g of DL-tert-leucine amide (0.093 mole), which was dissolved in 44.31 g of water. To the aqueous solution was added 3.3 g of acetic acid (0.056 mole), and the mixture was stirred. At this time, the pH of the mixture was 8.3. In addition, 5.97 mg of manganese chloride tetrahydrate was added to the mixture to prepare the stock solution of the reaction. After a 10.0 g portion of the stock solution was placed in a test tube, 0.24 g of the concentrated cell solution obtained in Referential Example 1 was inoculated and the mixture was subjected to stereoselective hydrolysis with stirring at 40°C. A 2.4 g pack of the molded tablets of Zeolite SAPO-34 (prepared according to Example 6 described in Japanese Patent Laid-Open Publication No. 2004-043296) in unwoven fabric was placed in the test tube so as the fabric to be immersed into the reaction solution.

The progress of the reaction was confirmed by the high performance liquid chromatography (HPLC) condition 1. The result of the reaction is illustrated in Figure 4. The rate of reaction in Figure 4 refers to the percentage of L-tert-leucine amide converted into L-tert-leucine in the raw material DL-tert-leucine amide. After 45 hours of reaction, 99.6% of L-tert-leucine amide was converted into L-tert-leucine. The sum of the ammonia concentration and the ammonium ion concentration reached 6000 ppm after 20 hours of reaction and was always maintained at this level or less by removing ammonia from the reaction system.

A filtrate from which the cells and the zeolite had been removed by filtering the reaction solution having active carbon and a filter aid added thereto was obtained. The mixture of the filtrate with 35 g of 2-methyl-1-propanol was subjected to azeotropic dehydration until the hydrated concentration reached 1% under 20 kPa for solvent displacement operation. The azeotropic dehydration proceeded at a boiling point of 60°C, and the boiling point reached 76°C at the completion of azeotropic dehydration. L-tert-leucine crystallized by solvent displacement was collected by suction filtration, washed with 25 g of 2-methyl-1-propanol warmed to 70°C and further with 75 g of acetone at 25°C, and subjected to vacuum drying at ambient temperature to give 5.9 g of white powder. Analysis of the L-tert-leucine according to the HPLC condition 1 revealed that the product had a chemical purity of 99.5%. The optical purity of the product measured according to the HPLC condition 2 was 99.5% or more.

### Comparative Example C-1

Under the same condition as in Example C-1, the reaction was conducted in the absence of zeolite. The result of the reaction is illustrated in Figure 4. After 46 hours of reaction, 68.5% of L-tert-leucine amide was converted into L-tert-leucine, and the rest remained as L-tert-leucine amide. The sum of the ammonia concentration and ammonium ion concentration in the reaction solution was increased with the progress of the reaction until 46 hours and reached the maximum of 8000 ppm.

### Comparative Example C-2

The reaction was conducted in the same manner as in Example 1 except that the suspension of the powdered zeolite SAPO-34 was added to the stereoselective enzymatic reaction solution. The result of the reaction is illustrated in Figure 4. After 46 hours of reaction, 3.6% of L-tert-leucine amide was converted into L-tert-leucine, and the rest remained as L-tert-leucine amide. The sum of the ammonia concentration and ammonium ion concentration in the reaction solution was increased with the progress of the reaction until 46 hours and reached the maximum of 400 ppm.

### Comparative Example C-3

To a 200 ml flask was added 20.0 g of DL-tert-leucine amide (0.15 mole), which was dissolved in 78.4 g of water. To the aqueous solution was added 1.86 g of acetic acid (0.031 mole), and the mixture was stirred. At this time, the pH of the mixture was 8.3. In addition, 6.88 mg of manganese chloride tetrahydrate and then 0.60 g of the concentrated cell solution obtained in Referential Example 1 were added and the mixture was subjected to stereoselective hydrolysis with stirring at 40°C in the absence of zeolite. The result of the reaction is illustrated in Figure 4. After 45 hours of reaction, 47.4% of L-tert-leucine amide was converted into L-tert-leucine and the rest remained as L-tert-leucine amide. The sum of the ammonia concentration and the ammonium ion concentration was increased with the progress of the reaction until 45 hours and reached the maximum of 7800 ppm.

### Example D: Enzymatic reaction with an immobilized biocatalyst under reduced pressure

### Example D-1: Enzymatic reaction with an immobilized biocatalyst under reduced pressure reduced pressure (40 mmHg)

To 600.2 g of DL-tert-leucine amide (4.61 mole) dissolved in 4642.8 g of water was added 55.4 g of acetic acid (0.92 mole), and the mixture was stirred. At this time, the pH of the mixture was 8.3. In addition, 0.32 g of manganese chloride tetrahydrate was added and the mixture was regarded as a substrate solution. To 200 g of the substrate solution in 500 ml flask was added 14.4 g of the immobilized biocatalyst prepared in Referential Example 2, and the mixture was subjected to stereoselective hydrolysis with stirring at 40°C under the pressure of 40 mmHg.

The whole amount of the reaction solution was taken out after 24 hours of reaction, and the surface of the immobilized biocatalyst and the flask were washed with pure water. To the mixture was added again 200 g of the substrate solution, and stereoselective hydrolysis was repeated with stirring at 40°C under the pressure of 40 mmHg.

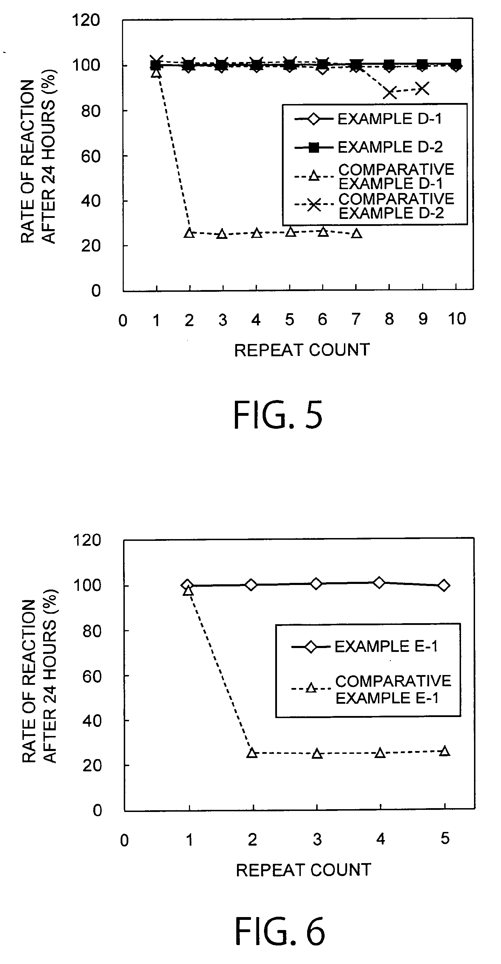

The rate of reaction after 24 hours measured with the HPLC analytical condition 1 is illustrated in Figure 5. The rate of reaction in Figure 5 refers to the percentage of L-tert-leucine amide converted into L-tert-leucine in the raw material DL-tert-leucine amide. As the result of 10 repeated reactions with the same immobilized biocatalyst, 99.0% or more of L-tert-leucine amide was converted into L-tert-leucine after 24 hours in all reactions. Moreover, the measurement after 24 hours of reaction with the HPLC analytical condition 2 revealed that L-tert-leucine had an optical purity of 100% ee in all reactions. The sum of the ammonia concentration and the ammonium ion concentration reached 2000 ppm after 6 hours of reaction and was always maintained at this level or less by removing ammonia from the reaction system.

### Example D-2: Enzymatic reaction (provided with ammonia separating apparatus) with immobilized biocatalyst under reduced pressure (40 mmHg)

### 1) Experimental apparatus

The apparatus is schematically illustrated in Figure 2.
The reaction solution in the reaction solution reservoir 1 is temperature controlled with the heater 2 and exposed to the atmosphere. At the same time, a portion of the reaction solution is sent to the reactor and evaporator 4 as the ammonia separating apparatus having the immobilized biocatalyst filled therein through the transfer pump of the reaction solution 3. The reactor and evaporator 4 is led to reduced pressure by the vacuum pump and temperature controlled. The reaction solution is subjected to reaction in the presence of the immobilized biocatalyst to remove ammonia, and returned to the reaction solution reservoir 1 through the transfer pump of the reaction solution 5. Furthermore, water can be added to the reaction solution reservoir 1 through the water supply line 6 during the reaction.

### 2) Stereoselective hydrolysis

To 600.5 g of DL-tert-leucine amide (4.61 mole) dissolved in 2357.7 g of water was added 56.2 g of acetic acid (0.94 mole), and the mixture was stirred. At this time, the pH of the mixture was 8.3. In addition, 0.32 g of manganese chloride tetrahydrate was added and the mixture was regarded as a substrate solution. In a 500 ml flask was filled 200 g of the substrate solution. 29.6 g of the immobilized biocatalyst prepared in Referential Example 2 was filled in the reactor and the evaporator, and the mixture was set at 40°C under the pressure of 40 mmHg. A portion of the substrate solution was circulated at a flow rate of 30 ml/min for stereoselective hydrolysis and the removal of ammonia. The amount of water decreased by evaporation is supplied from the water supply line.

The whole amount of the reaction solution was taken out after 24 hours of reaction, and the surfaces of the flask and the immobilized biocatalyst were washed with pure water by circulating it through the flask and the reactor and evaporator. To the mixture was added again 200 g of the substrate solution, and stereoselective hydrolysis was repeated.

The rate of reaction after 24 hours measured with the HPLC analytical condition 1 is illustrated in Figure 5. As the result of 10 repeated reactions with the same immobilized biocatalyst, 99.0% or more of L-tert-leucine amide was converted into L-tert-leucine after 24 hours in all reactions.

Moreover, the measurement after 24 hours of reaction with the HPLC analytical condition 2 revealed that L-tert-leucine had an optical purity of 100% ee in all reactions. The sum of the ammonia concentration and the ammonium ion concentration reached 3000 ppm after 6 hours of reaction and was always maintained at this level or less by removing ammonia from the reaction system.

### Comparative Example D-1

Stereoselective hydrolysis was conducted in the same manner as in Example D-1, except that the reaction was conducted under atmospheric pressure. The rate of reaction after 24 hours with the HPLC analytical condition 1 is illustrated in Figure 5. The rate of reaction in Figure 5 refers to the percentage of L-tert-leucine amide converted into L-tert-leucine in the raw material DL-tert-leucine amide. In the first reaction, 96.9% of L-tert-leucine amide was converted into L-tert-leucine after 24 hours. On the other hand, the reaction rate remained less than 27% after 24 hours, and thus the immobilized biocatalyst could not be used again.
In this connection, the sum of the ammonia concentration and the ammonium ion concentration was increased with the progress of the reaction until 24 hours and reached the maximum of 7000 ppm.

### Comparative Example D-2

In the same manner as in Example D-2, 9 stereoselective hydrolysis reactions were conducted repeatedly. In the seventh reaction, stereoselective hydrolysis was conducted under the atmospheric pressure of the reactor and evaporator 4 after 7.5 hours - 22.5 hours. In the sequential 8th and 9th reactions, stereoselective reactions were conducted with the reactor and evaporator 4 set at the pressure of 40 mmHg in the same manner as in Example 2. While the sum of the ammonia concentration and ammonium ion concentration in the 1st - 6th as well as 8th and 9th reaction solutions remained 3500 ppm at most, it reached 4500 ppm maximally at the seventh reaction. The rate of reaction after 24 hours with the HPLC analytical condition 1 is illustrated in Figure 5. In the 1st - 7th reactions, 99.5% or more of L-tert-leucine amide was converted into L-tert-leucine after 24 hours. On the other hand, the reaction rate remained less than 89% after 24 hours on and after the 8th reaction, and thus the immobilized biocatalyst could not be used again.

### Example E: Adsorption on cation exchange resin/enzymatic reaction with immobilized biocatalyst

### Example E-1

To 600.2 g of DL-tert-leucine amide (4.61 mole) dissolved in 4642.8 g of water was added 55.4 g of acetic acid (0.92 mole), and the mixture was stirred. At this time, the pH of the mixture was 8.3. In addition, 0.32 g of manganese chloride tetrahydrate was added and the mixture was regarded as a substrate solution. To 10.0 g of the substrate solution in a test tube was suspended 2.5 g of the cation exchange resin DOWEX 50WX8 (Dow Chemical), followed by 2.9 g of the immobilized biocatalyst prepared in Referential Example 2, and the mixture was subjected to stereoselective hydrolysis with stirring at 40°C.

The whole amount of the reaction solution was taken out after 24 hours of reaction, and the immobilized biocatalyst was separated. After the surface of the immobilized biocatalyst and the test tube were washed with pure water, 10.0 g of the substrate solution and 2.6 g of the cation exchange resin were added again to the mixture, and stereoselective hydrolysis was repeated with stirring at 40°C.

The rate of reaction after 24 hours measured with the HPLC analytical condition 1 is illustrated in Figure 6. The rate of reaction in Figure 6 refers to the percentage of L-tert-leucine amide converted into L-tert-leucine in the raw material DL-tert-leucine amide. As the result of 5 repeated reactions with the same immobilized biocatalyst, 99.0% or more of L-tert-leucine amide was converted into L-tert-leucine after 24 hours in all reactions.

Moreover, the measurement after 24 hours of reaction with the HPLC analytical condition 2 revealed that L-tert-leucine had an optical purity of 100% ee in all reactions. The sum of the ammonia concentration and the ammonium ion concentration reached the maximum of 2000 ppm after 6 hours of reaction and was always maintained at this level or less by removing ammonia from the reaction system.

A filtrate from which the cells had been removed by filtering the reaction solution having active carbon and a filter aid added thereto was obtained. The mixture of the filtrate with 10 g of 2-methyl-1-propanol was subjected to azeotropic dehydration until the hydrated concentration reached 1% under 20 kPa for solvent displacement operation. The azeotropic dehydration proceeded at a boiling point of 60°C, and the boiling point reached 76°C at the completion of azeotropic dehydration. L-tert-leucine crystallized by solvent displacement was collected by suction filtration, washed with 5 g of 2-methyl-1-propanol warmed to 70°C and further with 15 g of acetone at 25°C, and then subjected to vacuum drying at ambient temperature to give 0.96 g of white powder. Analysis of the L-tert-leucine according to the HPLC condition 1 revealed that the product had a chemical purity of 99.0%. The optical purity of the product measured according to the HPLC condition 2 was 99.5% or more.

### Comparative Example E-1

Stereoselective hydrolysis was conducted in the same manner as that in Example E-1 except that no ion exchange resin was added. The rate of reaction after 24 hours of reaction with the HPLC analytical condition 1 is illustrated in Figure 6. In the 1st reaction, 96.9% of L-tert-leucine amide was converted into L-tert-leucine after 24 hours of reaction. On the other hand, the reaction rate remained less than 27% after 24 hours on and after the second reaction, and thus the immobilized biocatalyst could not be used again.

In this connection, the sum of the ammonia concentration and the ammonium ion concentration in the first reaction solution was increased with the progress of the reaction until 24 hours and reached the maximum of 7000 ppm.

### Example F: Adsorption on zeolite/enzymatic reaction with immobilized biocatalyst

### Example F-1

To 600.2 g of DL-tert-leucine amide (4.61 mole) dissolved in 4642.8 g of water was added 55.4 g of acetic acid (0.92 mole), and the mixture was stirred. At this time, the pH of the mixture was 8.3. In addition, 0.32 g of manganese chloride tetrahydrate was added and the mixture was regarded as a substrate solution. A 10.0 g portion of the substrate solution was placed in a test tube. A 2.4 g pack of the molded tablets of Zeolite SAPO-34 (prepared according to Example 6 described in Japanese Patent Laid-Open Publication No. 2004-043296) in unwoven fabric was placed in the test tube so as the fabric to be immersed into the reaction solution. In addition, 2.9 g of the immobilized biocatalyst prepared in Referential Example 2 was added, and the mixture was subjected to stereoselective hydrolysis with stirring at 40°C. The whole amount of the reaction solution was taken out after 24 hours of reaction, and the immobilized biocatalyst was separated. After the surface of the immobilized biocatalyst and the test tube were washed with pure water, 2.4 g of the zeolite packed in the unwoven fabric was placed in the test tube so as the fabric to be immersed into the reaction solution. In addition, 10.0 g of the substrate solution was added again to the mixture, and stereoselective hydrolysis was repeated with stirring at 40°C.

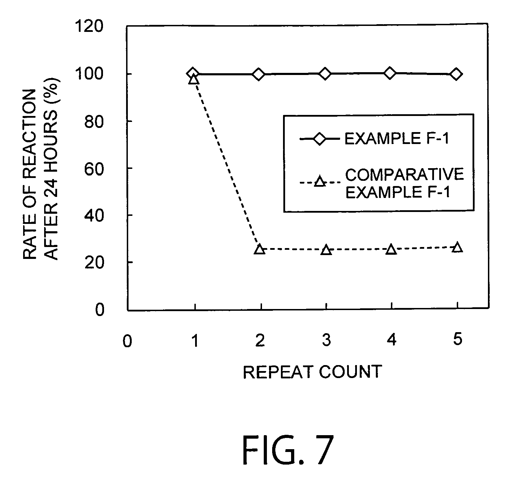

The rate of reaction after 24 hours measured with the HPLC analytical condition 1 is illustrated in Figure 7. The rate of reaction in Figure 7 refers to the percentage of L-tert-leucine amide converted into L-tert-leucine in the raw material DL-tert-leucine amide. As the result of 5 repeated reactions with the same immobilized biocatalyst, 99.0% or more of L-tert-leucine amide was converted into L-tert-leucine after 24 hours in all reactions.

Moreover, the measurement after 24 hours of reaction with the HPLC analytical condition 2 revealed that L-tert-leucine had an optical purity of 100% ee in all reactions. The sum of the ammonia concentration and the ammonium ion concentration reached the maximum of 2000 ppm after 6 hours of reaction and was always maintained at this level or less by removing ammonia from the reaction system.

A filtrate from which the cells and the zeolite had been removed by filtering the reaction solution having active carbon and a filter aid added thereto was obtained. The mixture of the filtrate with 35 g of 2-methyl-1-propanol was subjected to azeotropic dehydration until the hydrated concentration reached 1% under 20 kPa for solvent displacement operation. The azeotropic dehydration proceeded at a boiling point of 60°C, and the boiling point reached 76°C at the completion of azeotropic dehydration. L-tert-leucine crystallized by solvent displacement was collected by suction filtration, washed with 25 g of 2-methyl-1-propanol warmed to 70°C and further with 75 g of acetone at 25°C, and then subjected to vacuum drying at ambient temperature to give 5.9 g of white powder. Analysis of the L-tert-leucine according to the HPLC condition 1 revealed that the product had a chemical purity of 99.5%. The optical purity of the product measured according to the HPLC condition 2 was 99.5% or more.

### Comparative Example F-1

Stereoselective hydrolysis was conducted in the same manner as that in Example F-1 except that no zeolite was added. The rate of reaction after 24 hours of reaction with the HPLC analytical condition 1 is illustrated in Figure 7. In the first reaction, 96.9% of L-tert-leucine amide was converted into L-tert-leucine after 24 hours of reaction. On the other hand, the reaction rate remained less than 27% after 24 hours on and after the second reaction, and thus the immobilized biocatalyst could not be used again.

In this connection, the sum of the ammonia concentration and the ammonium ion concentration in the first reaction solution was increased with the progress of the reaction until 24 hours and reached the maximum of 7000 ppm.

### DESCRIPTION OF SYMBOLS IN THE DRAWINGS

- 1: Reaction vessel or reaction solution reservoir
- 2: Heater
- 3: Transfer pump of reaction solution
- 4: Evaporator or reactor and evaporator
- 5: Transfer pump of reaction solution
- 6: Water supply line

## Claims

1. A method for producing D- or L-tert-leucine or D- or L-tert-leucine amide by reacting DL-tert-leucine amide with a biocatalyst selected from the group consisting of an enzyme capable of hydrolyzing the DL-tert-leucine amide stereoselectively, cells of a microorganism having the enzyme, a material produced by the treatment of the cells, an immobilized enzyme produced by immobilizing the enzyme onto a carrier, immobilized cells produced by immobilizing the cells onto a carrier, and an immobilized cell treatment product obtained by immobilizing the cell treatment product onto a carrier to thereby hydrolyze the DL-tert-leucine amide,
wherein the hydrolysis is carried out with separating ammonia produced by the hydrolysis from the hydrolysis reaction solution.

2. A method according to claim 1, wherein ammonia is separated from the reaction solution by placing the reaction solution under reduced pressure to evaporate the ammonia or by having the ammonia in the reaction solution adsorbed on a cation exchange resin or zeolite.

3. A method according to claim 1, wherein ammonia is separated from the reaction solution by placing the reaction solution under reduced pressure to evaporate the ammonia.

4. A method according to claim 1, wherein ammonia is separated from the reaction solution by having the ammonia in the reaction solution adsorbed on a cation exchange resin.

5. A method according to claim 1, wherein ammonia is separated from the reaction solution by adsorbing an optically active amino acid or an optically active amino acid amide in the reaction solution on zeolite.

6. A method according to any one of claims 1 to 5, wherein the enzyme is the one derived from Xanthobacter flavus.

7. A method according to any one of claims 1 to 5, wherein the microorganism is the one derived from Xanthobacter flavus and having the gene of an enzyme capable of hydrolyzing the DL-tert-leucine amide stereoselectively introduced therein.

8. A method according to any one of claims 1 to 5, wherein the microorganism is pMCA1/JM109 (FERM BP-10334).

9. A method according to any one of claims 1 to 8, wherein the sum of the concentrations of ammonia and an ammonium ion in the reaction solution is in the range of 7000 ppm or less.
